# FASCICULE DE BREVET EUROPEEN

(11) **EP 0 682 003 B1**
(45) Date de publication et mention de la délivrance du brevet: **22.09.1999**
(21) Numéro de dépôt: 95460022.7
(22) Date de dépôt: 09.05.1995
(51) Int. Cl.: C07C 29/78, C07C 31/26

(54) **Procédé de production d'un extrait concentré liquide de végétaux à partir de végétaux renfermant du mannitol, extrait et mannitol ainsi obtenus**
Verfahren zur Herstellung eines flüssigen konzentrierten Pflanzenextraktes aus mannitolhaltigen Pflanzen und auf diese Weise hergestellter Extrakt und Mannitol
Process for producing a liquid concentrated plant extract from plants containing mannitol and thus obtained extract and mannitol

(30) Priorité: 11.05.1994 FR 9406033
(43) Date de publication de la demande: 15.11.1995
(73) Titulaire: AROMES DE BRETAGNE, F-35650 Antrain-sur-Couesnon (FR)
(72) Inventeur: Megard, Denis, F-35460 Saint Brice en Cogles (FR)
(74) Mandataire: Le Faou, Daniel

(56) Documents cités:
- EP-A- 0 202 168
- AU-A- 3 771 172
- US-A- 2 723 295
- US-A- 2 768 980
- US-A- 3 632 656

## Description

La présente invention concerne un procédé de production d'un extrait concentré liquide de végétaux à faible viscosité et de bonne qualité gustative, appauvri en mannitol, à partir de végétaux renfermant du mannitol.

Par les termes "végétal" et "végétaux", on entendra dans l'ensemble de la présente description et des revendications, les légumes ainsi que les champignons.

Ce procédé est particulièrement adapté à la préparation d'un extrait de champignons de Paris, ce produit étant naturellement riche en mannitol.

L'invention concerne également l'extrait obtenu par la mise en oeuvre du procédé.

La production d'un extrait aromatique concentré de champignon de Paris (Agaricus bisporus) fait intervenir les étapes suivantes : macération de la matière première ; extraction solide/liquide ; filtration et clarification du jus ; concentration à basse température, c'est-à-dire inférieure à 80° C, sous vide.

Ces étapes sont celles que l'on utilise aussi pour la fabrication des concentrés liquides de légumes ou de fruits.

L'extrait de champignon ne peut être concentré à plus de 30°Brix sans qu'il y ait cristallisation d'une partie de l'extrait lors de son refroidissement, avec un phénomène de prise en masse à partir de 55-60°Brix. Cette cristallisation, associée à la prise en masse, rend impossible la commercialisation d'un extrait titrant plus de 65°Brix.

Ainsi, un extrait concentré à 30°Brix est fluide à 4°C, n'est pas sujet à une prise en masse et ne contient pas de cristaux. Par contre, un extrait concentré à 60°Brix est pâteux à 4°C, prend en masse et contient environ 30% en poids de cristaux.

Entre 30 et 50°Brix, la phase cristallisée se dépose dans le fond des récipients par décantation naturelle et tend à former un agrégat cristallin dur dans le temps, tandis qu'à partir et au-dessus de 50°Brix, la viscosité élevée du milieu conduit à une répartition homogène des cristaux dans la masse et le produit devient une pâte épaisse difficile à manipuler et utiliser.

Le phénomène de cristallisation et de prise en masse est favorisé par le stockage du produit au froid (4°C) mais se produit, moins rapidement, lors du stockage de l'extrait à température ambiante (20°C).

L'analyse de la composition d'un extrait pâteux concentré à 65°Brix (55 à 60 % de matière sèche totale) donne les résultats suivants, comparés à la composition moyenne du champignon de Paris frais.

| | **Extrait concentré à 55°Brix (% poids)** | **Champignon frais (% poids)** |
|---|---|---|
| eau | 44,0 | 92,1 |
| protéines (N x 6,25) | 16,6 | 2,8 |
| matière grasses | 0,1 | 0,3 |
| minéraux | 18,4 | 1,2 |
| fibres | - | 2,0 |
| sucres réducteurs * | 0,9 | 0,4 |
| mannitol | 20,0 | 1,2 |

| | | |
|---|---|---|
| * sucres réducteurs = glucose, fructose, saccharose | | |

Une analyse plus fine des cristaux par spectrométrie de masse a montré que le composé qui cristallise est le mannitol, ce qui est confirmé par la faible solubilité du mannitol dans l'eau (182 g/l à 25°C).

Par conséquent, dès lors qu'on se propose de fabriquer un extrait fortement concentré, ce qui correspond à une demande importante de la part des utilisateurs de tels extraits, on se heurte à la difficulté qui réside dans le phénomène de cristallisation du mannitol au sein de l'extrait.

L'élimination du mannitol en solution dans des jus a été tentée, notamment par une voie biologique de dégradation de ce composé, ainsi que par des technologies membranaires. Malheureusement, cette élimination se traduit par une altération des qualités aromatiques, c'est-à-dire gustatives du produit final.

On décrit dans le document AU-D-3 771 172, un procédé de séparation de mannitol d'un mélange simple formé de mannitol et de sorbitol. Ce procédé consiste à traiter la solution de manière à former des cristaux de mannitol et à séparer le mannitol cristallin de la solution. Selon un autre aspect, ce document divulgue un procédé qui consiste à ensemencer une solution de mannitol / sorbitol avec les cristaux déjà produits et à récupérer le mannitol ainsi cristallisé. Dans les exemples décrits, on concentre la solution à une température comprise entre 80 et 107° C et la cristallisation est effectuée

entre 53 et 65° C pendant deux à plusieurs heures. Une telle méthodologie n'est pas transposable à un composé complexe que constitue un extrait liquide de végétaux dans la mesure où la chaleur dégrade les molécules aromatiques et où il apparaît des réactions dites "de Maillard" qui consistent en des associations sucres protéines, responsables de brunissement et de goûts de cuit.

Les mêmes commentaires s'appliquent au document EP-A-202 168 qui concerne un procédé et une installation de cristallisation de mannitol.

Deux autres documents décrivent des procédés de séparation de mélanges simples.

Il s'agit du document US-A-2 768 980 relatif à un procédé de cristallisation de mannitol, à partir d'un mélange formé de mannitol et d'un polyol. Après ajustement du pH à 3,5, la solution est refroidie à environ 18-20° sous agitation. Le mannitol est séparé après cristallisation.

Il s'agit également du document 2 723 951. On y décrit un procédé de cristallisation de mannitol à partir d'un mélange formé de mannitol et de sorbitol. Après concentration par évaporation, la cristallisation du mélange est réalisée selon un gradient de température d'un degré à l'heure, jusqu'à obtention d'une température de 20° C. On ne connait ni la température de départ, ni la durée de refroidissement.

L'invention vise à résoudre ce problème. En d'autres termes, elle vise à fournir un procédé de production d'un extrait concentré liquide de végétaux par élimination de mannitol, cet extrait étant de faible viscosité et de qualité gustative ou aromatique améliorée par rapport à celle d'un extrait traditionnel.

Cet objectif est atteint conformément à l'invention par le fait que ce procédé consiste à :
a) former un extrait concentré liquide desdits végétaux titrant entre 50 et 65°Brix, la concentration étant réalisée à une température inférieure à 80° C, sous vide.
b) provoquer la cristallisation à froid du mannitol au sein dudit extrait ;
c) éliminer de la phase liquide la phase cristallisée renfermant le mannitol.

Ce procédé permet donc d'enlever le mannitol de l'extrait après cristallisation, ce qui est délicat en raison de la forte viscosité du milieu. En effet, un concentré à 65°Brix présente une viscosité de 1000 à 6000 mPa.s à 10°C, selon la force de cisaillement appliquée, avec un comportement thixotrope et rhéofluidifiant, ce qui signifie qu'il se liquéfie lorsqu'il est agité et reprend sa viscosité initiale au repos.

Après traitement, l'extrait obtenu présente une viscosité deux à trois fois plus faible (dans les mêmes conditions de température et de force de cisaillement).

De plus, les qualités aromatiques de l'extrait faiblement visqueux obtenu sont améliorées par rapport à l'extrait concentré brut. La puissance aromatique augmente d'autant puisqu'on enlève le mannitol qui est un polyol à goût sucré.

Selon d'autres caractéristiques avantageuses et non limitatives de ce procédé :
- à l'étape a), on réalise la concentration pendant une durée inférieure ou égale à 30 minutes, et plus préférentiellement égale à 15 minutes ;
- à l'étape a), on forme un extrait titrant entre 60 et 65°Brix ;
- à l'étape b), on réalise la cristallisation sous agitation ;
- on réalise la cristallisation à une température comprise entre 0 et 10°C et pendant une durée comprise entre 1 et 72 heures ;
- on réalise la cristallisation à température ambiante et pendant une durée supérieure ou égale à 24 heures ;
- on réalise l'étape c) au moyen d'un séparateur solide/liquide tel qu'une essoreuse ou un filtre presse ;
- on réalise l'étape c) à une température comprise entre 5 et 40°C, et de préférence inférieure à 25°C ;
- à l'issue de l'étape c), on mélange la phase cristallisée obtenue avec de l'eau et l'on renouvelle les étapes b) et c) sur ce mélange ;
- on répète plusieurs fois cette opération sur la phase cristallisée obtenue ;
- on rassemble les différentes phases liquides obtenues et éventuellement on les concentre ;
- à l'issue de l'étape c), on récupère la phase cristallisée et, éventuellement, on la purifie.

L'invention concerne également l'extrait concentré obtenu par la mise en oeuvre d'un tel procédé.

On notera que la première étape du procédé, qui consiste à former un extrait concentré liquide de végétaux titrant entre 50 et 65°Brix, est mise en oeuvre selon une méthode bien connue de l'homme du métier. Elle est réalisée sous vide, à une température inférieure à 80° C.

De préférence, la concentration est réalisée pendant une durée inférieure ou égale à 30 minutes et encore plus préférentiellement égale à 15 minutes. Une durée de traitement supérieure à 30 minutes aurait pour inconvénient d'abîmer une partie des molécules aromatiques.

La concentration de l'extrait située entre 50 et 65°Brix, et plus préférentiellement 60 à 65°Brix, permet d'obtenir le maximum de mannitol sous forme insoluble.

La cristallisation de l'extrait peut être menée en cuve agitée (cristallisoir), à froid, entre 0 et 10°C pendant une durée de l'ordre de 2 à 72 heures ou à température ambiante sur une durée de 24 heures à plusieurs jours.

Lorsque la cristallisation est réalisée, l'extrait est transféré, par exemple par pompage jusqu'à un système de séparation solide/liquide, qui peut consister en une essoreuse ou un filtre presse. La porosité des toiles ou filtres de ces équipements est avantageusement comprise entre 100 et 500 micromètres. La séparation peut être réalisée à une température comprise entre 5 et 40°C et plus préférentiellement en-dessous de 25°C afin que le mannitol reste insoluble.

On peut bien entendu utiliser tout autre type de séparateur solide/liquide compatible avec la viscosité de l'extrait.

A titre indicatif, on peut utiliser une essoreuse telle que celles commercialisées par la société ROUSSELET ou un filtre presse de la société LAROX ou CHOQUENET.

La cristallisation peut par ailleurs être réalisée dans des récipients non soumis à une agitation.

L'extrait débarrassé de mannitol est éventuellement standardisé, c'est-à-dire mélangé avec d'autres concentrés présentant sensiblement la même concentration, ceci afin d'obtenir un produit final de qualité constante.

Bien entendu, cet extrait peut être stérilisé et conditionné aseptiquement.

La phase cristallisée formée essentiellement de mannitol peut être mélangée à de l'eau et soumise à nouveau à une cristallisation afin d'obtenir une phase liquide éventuellement incorporable dans l'extrait concentré.

On trouvera ci-dessous la description d'un exemple de fabrication d'un extrait de champignon de Paris, conformément au procédé de l'invention.

### Exemple : Traitement d'un extrait concentré de champignons de Paris

On fabrique 1000 kg d'un concentré E₀ titrant 55°Brix (45 à 50 % de matière sèche totale) selon la méthode citée dans l'introduction de la présente description.

On soumet cet extrait E₀ à une cristallisation en le transférant dans un cristalloir pourvu de moyens d'agitation (pales). Cette opération est réalisée à température ambiante (environ 20°C) et pendant une durée de 24 heures.

On soumet alors l'extrait à un essorage pendant une durée de trois minutes. On obtient alors 510 kg d'un concentré fluide titrant 55°Brix (extrait E₁) et 490 kg de cristaux formant une pâte noire (C₁).

On mélange alors la masse C₁ à 385 kg d'eau et on réalise une nouvelle cristallisation dans les mêmes conditions que précédemment, puis on essore.

On récupère alors 435 kg d'un concentré liquide titrant 30°Brix (extrait E₂) et 426 kg de cristaux bruns clairs (C₂).

L'extrait E₂ est concentré jusqu'à obtention de 230 kg d'un extrait titrant 55°Brix (extrait E'₂). E'₂ est alors mélangé à E₁ de sorte qu'on recueille 740 kg d'un extrait concentré liquide à faible viscosité, titrant 55°Brix et possédant une puissance aromatique 30 % supérieure à celle de E₀. Par "puissance", on entend la force aromatique estimée par des tests organoleptiques comparatifs à dilution identique.

On mélange la masse C₂ avec 184 kg d'eau et on opère une cristallisation et un essorage. On obtient 264 kg d'un concentré titrant 25°Brix (E₃) et 332 kg de cristaux beiges (C₃).

L'extrait E₃ est concentré pour obtenir 125 kg d'un concentré titrant 52°Brix (E'₃). Ce concentré est visqueux et contient des cristaux. Il se fluidifie sous agitation et présente une puissance aromatique inférieure de 80 % à celle de l'extrait E₀. Il présente une note beaucoup plus sucrée.

La masse C₃ est mélangée à 143 kg d'eau. On opère alors une cristallisation et un essorage. On obtient 197 kg d'un concentré titrant 17°Brix (E₄) et 260 kg de cristaux bruts de couleur blanc cassé (C₄).

Après concentration de E₄, on obtient 115 kg d'un concentré titrant 28°Brix. Il s'agit d'un concentré pris en masse ayant un goût de sucre et présentant des traces de champignon. E₄ et E₃ peuvent être éventuellement réunis et incorporés dans un nouvel extrait E₀ à traiter.

Après séchage à l'air chaud de C₄, on obtient 200 kg de cristaux de mannitol à 99,8 % de pureté.

On notera que la concentration de E₂ avant son mélange avec E₁ peut être évitée si on souhaite commercialiser un extrait titrant environ 40°Brix. Bien entendu, si l'on traite un extrait E₀ plus fortement concentré (par exemple 65°Brix), les extraits obtenus n'ont pas nécessairement besoin d'être à nouveau concentrés. Par ailleurs, les différents extraits peuvent être commercialisés tels quels, sans avoir été rassemblés avec d'autres extraits.

Les légères pertes de produit après essorage (inférieures à 5 % de la masse mise en oeuvre) sont dues aux différentes manipulations.

La durée et la vitesse d'essorage peuvent être variables. En particulier, la vitesse dépend de l'outil utilisé (limites mécaniques) et de la résistance des toiles ou filtres utilisés. Leur détermination est à la portée de l'homme du métier.

Dans l'exemple décrit ci-dessus, le second essorage et les suivants ont une durée variant entre 3 et 30 minutes.

On notera qu'au dessous de 55°Brix, la commercialisation des extraits nécessite une pasteurisation et un conditionnement aseptique car ils sont microbiologiquement instables.

Enfin, on notera que les étapes de cristallisation peuvent être de plus courte durée, ce qui se traduit par le fait qu'une partie du mannitol reste en solution. Si l'on souhaite débarasser totalement l'extrait du mannitol qu'il contient, il y aura lieu de renouveler un nombre élevé de fois celle étape de cristallisation.

L'invention permet donc non seulement d'obtenir des extraits concentrés à faible viscosité et de bonne qualité aromatique mais également du mannitol. En effet, jusqu'à maintenant le mannitol est produit par voie biotechnologique (utilisation d'enzymes) à partir de sirops de fructose.

Le procédé qui vient être décrit peut être mis en oeuvre pour d'autres végétaux renfermant du mannitol.

## Revendications

1. Procédé de production d'un extrait concentré liquide de végétaux à faible viscosité et de qualité aromatique améliorée, appauvri en mannitol, à partir de végétaux renfermant du mannitol qui consiste à :
a) former un extrait liquide desdits végétaux titrant entre 50 et 65°Brix, la concentration étant réalisée à une température inférieure à 80° C, sous vide ;
b) provoquer la cristallisation à froid du mannitol au sein dudit extrait ;
c) éliminer de la phase liquide la phase cristallisée renfermant le mannitol.

2. Procédé selon la revendication 1, caractérisée en ce que à l'étape a), on réalise la concentration pendant une durée inférieure ou égale à 30 minutes, et plus préférentiellement égale à 15 minutes.

3. Procédé selon la revendication 1 ou 2, caractérisé en ce que, à l'étape a), on forme un extrait titrant entre 60 et 65°Brix.

4. Procédé selon l'une des revendications 1 à 3, caractérisé en ce qu'à l'étape b), on réalise la cristallisation sous agitation.

5. Procédé selon la revendication 4, caractérisé en ce qu'on réalise la cristallisation à une température comprise entre 0 et 10°C et pendant une durée comprise entre 1 et 72 heures.

6. Procédé selon la revendication 4, caractérisé en ce qu'on réalise ladite cristallisation à température ambiante et pendant une durée supérieure ou égale à 24 heures.

7. Procédé selon l'une des revendications 1 à 6, caractérisé en ce qu'on réalise l'étape c) au moyen d'un séparateur solide/liquide tel qu'une essoreuse ou un filtre presse.

8. Procédé selon l'une des revendications 1 à 6, caractérisé en ce qu'on réalise l'étape c) à une température comprise entre 5 et 40°C, et de préférence inférieure à 25°C.

9. Procédé selon l'une des revendications 1 à 8, caractérisé en ce que, a l'issue de l'étape c), on mélange la phase cristallisée obtenue avec de l'eau et en ce que l'on renouvelle les étapes b) et c) sur ce mélange.

10. Procédé selon la revendication 9, caractérisé en ce qu'on répète plusieurs fois cette opération sur la phase cristallisée obtenue.

11. Procédé selon la revendication 9 ou 10, caractérisée en ce que l'on rassemble les différentes phases liquides obtenues et en ce que, éventuellement on concentre celles-ci.

12. Procédé selon l'une des revendications 1 à 11, caractérisé en ce que, a l'issue de l'étape c), on récupère la phase cristallisée et en ce que, éventuellement, on la purifie.

13. Extrait concentré liquide de végétaux à faible viscosité et de qualité aromatique améliorée, obtenu par la mise en oeuvre du procédé selon l'une des revendications 1 à 11.

## Claims

1. A method of producing a concentrated mannitol-depleted liquid plant extract of low viscosity and improved aromatic quality from plants containing mannitol, the method consisting in:
a) forming, from said plants, a liquid extract titrating in the range 50°Brix to 65°Brix, with concentration being performed at a temperature below 80°C under a vacuum;
b) causing the mannitol to crystallize cold within said extract; and
c) removing the mannitol-containing crystals from the liquid.

2. A method according to claim 1, characterized in that in step a), concentration is performed for a period of time less than or equal to 30 minutes, and more preferably equal to 15 minutes.

3. A method according to claim 1 or 2, characterized in that in step a) an extract is formed that titrates in the range 60°Brix to 65°Brix.

4. A method according to any one of claims 1 to 3, characterized in that, in step b), crystallization is performed while stirring.

5. A method according to claim 4, characterized in that crystallization is performed at a temperature lying in the range 0°C to 10°C and for a duration lying in the range 1 hour to 72 hours.

6. A method according to claim 4, characterized in that said crystallization is performed at ambient temperature for a duration greater than or equal to 24 hours.

7. A method according to any one of claims 1 to 6, characterized in that step c) is performed by means of a solid-liquid separator such as a centrifuge or a filter press.

8. A method according to any one of claims 1 to 6, characterized in that step c) is performed at a temperature lying in the range 5°C to 40°C, and preferably less than 25°C.

9. A method according to any one of claims 1 to 8, characterized in that, at the end of step c), the resulting crystals are mixed with water, and in that steps b) and c) are performed again on the mixture.

10. A method according to claim 9, characterized in that said operation is repeated a plurality of times on the resulting crystals.

11. A method according to claim 9 or 10, characterized in that the various liquids obtained are put together, and in that they are optionally concentrated.

12. A method according to any one of claims 1 to 11, characterized in that, at the end of step c), the crystals are recovered and in that they are optionally purified.

13. A concentrated liquid plant extract having low viscosity and improved aromatic quality, obtained by implementing the method according to any one of claims 1 to 11.

## Patentansprüche

1. Verfahren zur Herstellung eines konzentrierten flüssigen Pflanzenextrakts mit geringer Viskosität und verbesserter Aromaqualität, mit verringertem Mannit-Gehalt, ausgehend von Pflanzen, die Mannit enthalten, welches darin besteht:
a) einen flüssigen Extrakt, der Pflanzen mit einem Titer von 50 bis 65°Brix, zu bilden, wobei das Konzentrieren bei einer Temperatur unter 80°C im Vakuum erfolgt;
b) die Kristallisation des Mannits aus dem Extrakt in der Kälte einzuleiten;
c) von der flüssigen Phase, die den Mannit enthaltende kristalline Phase zu entfernen.

2. Verfahren nach Anspruch 1, dadurch gekennzeichnet, daß man in der Stufe a) das Konzentrieren während einer Dauer unter oder gleich 30 Minuten und bevorzugter gleich 15 Minuten durchführt.

3. Verfahren nach Anspruch 1 oder 2, dadurch gekennzeichnet, daß man in der Stufe a) einen Extrakt mit einem Titer von 60 bis 65°Brix bildet.

4. Verfahren nach einem der Ansprüche 1 bis 3, dadurch gekennzeichnet, daß man in der Stufe b) die Kristallisation unter Bewegen durchführt.

5. Verfahren nach Anspruch 4, dadurch gekennzeichnet, daß man die Kristallisation bei einer Temperatur von 0 bis 10°C und während einer Dauer von 1 bis 72 Stunden durchführt.

6. Verfahren nach Anspruch 4, dadurch gekennzeichnet, daß man die Kristallisation bei Raumtemperatur und während einer Dauer von über oder gleich 24 Stunden durchführt.

7. Verfahren nach einem der Ansprüche 1 bis 6, dadurch gekennzeichnet, daß man die Stufe c) mit einem Feststoff/Flüssigkeits-Separator, wie einer Zentrifuge oder einer Filterpresse, durchführt.

8. Verfahren nach einem der Ansprüche 1 bis 6, dadurch gekennzeichnet, daß man die Stufe c) bei einer Temperatur von 5 bis 40°C, vorzugsweise unter 25°C, durchführt.

9. Verfahren nach einem der Ansprüche 1 bis 8, dadurch gekennzeichnet, daß man im Verlauf der Stufe c) die erhaltene kristalline Phase mit Wasser vermischt und daß man die Stufen b) und c) mit diesem Gemisch erneut durchführt.

10. Verfahren nach Anspruch 9, dadurch gekennzeichnet, daß man diese Verfahrensweise an der erhaltenen kristallinen Phase mehrfach wiederholt.

11. Verfahren nach Anspruch 9 oder 10, dadurch gekennzeichnet, daß man die verschiedenen erhaltenen flüssigen Phasen vereint und daß man diese gegebenenfalls konzentriert.

12. Verfahren nach einem der Ansprüche 1 bis 11, dadurch gekennzeichnet, daß man im Verlauf der Stufe c) die kristallisierte Phase gewinnt und daß man sie gegebenenfalls reinigt.

13. Konzentrierter flüssiger Pflanzenextrakt mit geringer Viskosität und verbesserter Aromaqualität, erhalten durch Durchführung des Verfahrens nach einem der Ansprüche 1 bis 11.
